# EUROPEAN PATENT SPECIFICATION

(11) **EP 2 844 763 B1**
(45) Date of publication and mention of the grant of the patent: **22.02.2017**
(21) Application number: 13720711.4
(22) Date of filing: 25.04.2013
(51) Int. Cl.: C12Q 1/68

(54) **IN VITRO METHOD FOR PREDICTING DISEASE OUTCOME IN STAGE II COLORECTAL CANCER**
IN-VITRO-VERFAHREN ZUR VORHERSAGE DES KRANKHEITSAUSGANGS IN PHASE II VON KOLOREKTALEM KREBS
PROCÉDÉ IN VITRO POUR PRÉDIRE L'ÉVOLUTION DE LA MALADIE DANS LE STADE II DU CANCER COLORECTAL

(30) Priority: 29.04.2012 EP 12166093; 30.04.2012 US 201261640540 P
(43) Date of publication of application: 11.03.2015
(73) Proprietor: Universiteit Maastricht, 6211 LK Maastricht (NL); Academisch Ziekenhuis Maastricht, 6229 HX Maastricht (NL); The Johns Hopkins University, Baltimore, MD 21218-2695 (US)
(72) Inventor: VAN ENGELAND, Manon, NL-6229 EV Maastricht (NL); DE BRUINE, Adriaan, Pieter, NL-6243 CN Geulle (NL); HERMAN, James, G., Baltimore, MD 21218-2695 (US)
(74) Representative: Habets, Winand
(86) International application number: PCT/US2013/038099
(87) International publication number: WO 2013/165788

(56) References cited:
- WO-A1-2007/110450
- WO-A1-2009/114836
- WO-A1-2009/137094
- M. TANAKA ET AL: "Association of CHFR Promoter Methylation with Disease Recurrence in Locally Advanced Colon Cancer", CLINICAL CANCER RESEARCH, vol. 17, no. 13, 1 July 2011 (2011-07-01), pages 4531-4540, XP055032672, ISSN: 1078-0432, DOI: 10.1158/1078-0432.CCR-10-0763
- KAZUYUKI KAWAKAMI ET AL: "Long interspersed nuclear element-1 hypomethylation is a potential biomarker for the prediction of response to oral fluoropyrimidines in microsatellite stable and CpG island methylator phenotype-negative colorectal cancer", CANCER SCIENCE, vol. 102, no. 1, 19 November 2010 (2010-11-19), pages 166-174, XP055032665, ISSN: 1347-9032, DOI: 10.1111/j.1349-7006.2010.01776.x
- SOFIA B. GUSTAFSSON ET AL: "High Tumour Cannabinoid CB1 Receptor Immunoreactivity Negatively Impacts Disease-Specific Survival in Stage II Microsatellite Stable Colorectal Cancer", PLOS ONE, vol. 6, no. 8, 25 August 2011 (2011-08-25) , page E23003, XP055032666, DOI: 10.1371/journal.pone.0023003
- R. L. WARD: "Adverse Prognostic Effect of Methylation in Colorectal Cancer Is Reversed by Microsatellite Instability", JOURNAL OF CLINICAL ONCOLOGY, vol. 21, no. 20, 8 September 2003 (2003-09-08), pages 3729-3736, XP055032670, ISSN: 0732-183X, DOI: 10.1200/JCO.2003.03.123
- SUNG Y K ET AL: "746 CpG Island Methylator Phenotype May Confer a Survival Benefit From Fluoropyrimidine-Based Adjuvant Chemotherapy on Patients With Stage II or III Colorectal Carcinomas", GASTROENTEROLOGY, ELSEVIER, PHILADELPHIA, PA, vol. 138, no. 5, 1 May 2010 (2010-05-01), pages S-102, XP027022993, ISSN: 0016-5085 [retrieved on 2010-04-27]
- S. TEJPAR ET AL: "Microsatellite Instability, Prognosis and Drug Sensitivity of Stage II and III Colorectal Cancer: More Complexity to the Puzzle", JNCI JOURNAL OF THE NATIONAL CANCER INSTITUTE, vol. 103, no. 11, 8 June 2011 (2011-06-08) , pages 841-844, XP055032664, ISSN: 0027-8874, DOI: 10.1093/jnci/djr170
- BRANDES JOHANN C ET AL: "CHFR promoter hypermethylation in colon cancer correlates with the microsatellite instability phenotype", CARCINOGENESIS, OXFORD UNIVERSITY PRESS, GB, vol. 26, no. 6, 1 June 2005 (2005-06-01), pages 1152-1156, XP002437999, ISSN: 0143-3334, DOI: 10.1093/CARCIN/BGI058
- S. DERKS ET AL: "Integrated analysis of chromosomal, microsatellite and epigenetic instability in colorectal cancer identifies specific associations between promoter methylation of pivotal tumour suppressor and DNA repair genes and specific chromosomal alterations", CARCINOGENESIS, vol. 29, no. 2, 28 November 2007 (2007-11-28), pages 434-439, XP055064442, ISSN: 0143-3334, DOI: 10.1093/carcin/bgm270

### SEQUENCE LISTING

<110> Universiteit Maastricht and Academisch Ziekenhuis Maastricht
<120> In vitro Method For Predicting Disease Outcome In Stage II Colorectal Cancer
<130> 190 EPP0
<160> 6
<170> PatentIn version 3.5
<210> 1
   <211> 31
   <212> DNA
   <213> homo sapiens
<400> 1
   ttttygtttt ttttgtttta atataatatg g 31
<210> 2
   <211> 26
   <212> DNA
   <213> homo sapiens
<400> 2
   crctcaccaa aaacracaac taaaac 26
<210> 3
   <211> 32
   <212> DNA
   <213> homo sapiens
<400> 3
   gattgtagtt atttttgtga tttgtaggtg at 32
<210> 4
   <211> 29
   <212> DNA
   <213> homo sapiens
<400> 4
   aactaaaaca aaaccaaaaa taacccaca 29
<210> 5
   <211> 25
   <212> DNA
   <213> homo sapiens
<400> 5
   gttattttcg tgattcgtag gcgac 25
<210> 6
   <211> 21
   <212> DNA
   <213> homo sapiens
<400> 6
   cgaaaccgaa aataacccgc g 21

## Description

### PRIORITY CLAIM

This application claims the benefit of the filing date of United States Provisional Patent Application Serial No. 61/640,540, filed April 30, 2012, and European Patent Application Serial No. 12166093.0, filed April 29, 2012.

### TECHNICAL FIELD

The present invention relates to a method for determining the likelihood of disease outcome of a patient diagnosed with microsatellite stable, stage II colorectal cancer.

### BACKGROUND

Accurate staging of colorectal cancer (CRC) is essential for optimal management of the disease. Although patients with the same stage can demonstrate considerable variation in disease outcome, the TNM staging system remains the gold standard for predicting prognosis and guiding clinical management of CRC (C.C. Compton and F.L. Greene, The staging of colorectal cancer: 2004 and beyond, CA: A Cancer Journal for Clinicians 54 (6):295-308, 2004).

The TNM Classification of Malignant Tumors (TNM) is a cancer staging system that describes the extent of cancer in a patient's body. T describes the size of the tumor and whether it has invaded nearby tissue, N describes regional lymph nodes that are involved, M describes distant metastasis (spread of cancer from one body part to another).

The TNM staging system for all solid tumors was devised by Pierre Denoix between 1943 and 1952, using the size and extension of the primary tumor, its lymphatic involvement, and the presence of metastases to classify the progression of cancer.

TNM is developed and maintained by the International Union Against Cancer (UICC) to achieve consensus on one globally recognized standard for classifying the extent of spread of cancer. The TNM classification is also used by the American Joint Committee on Cancer (AJCC) and the International Federation of Gynecology and Obstetrics (FIGO). In 1987, the UICC and AJCC staging systems were unified into a single staging system.

Colorectal cancer (CRC) accounts for 10%-15% of all cancers and is the leading cause of cancer deaths in the Western world. Up to 40%-50% of patients who undergo potentially curative surgery alone ultimately relapse and die of metastatic disease. The most important prognostic indicator for survival in colon cancer is tumor stage, which is determined by the depth of penetration through the bowel wall and the number of lymph nodes involved.

In stage II CRC, there is tumor penetration through the bowel wall involving the serosa; however, there is no involvement of regional lymph nodes or distant metastases. While the overall survival in this subgroup of patients is approximately 70%-80% 5 years after surgery, it has been recognized that there is a subgroup of patients with a high-risk stage II disease, in which the clinical outcome is similar to that of patients with stage III disease. Currently, these high-risk patients are identified by tumors that not only penetrate the bowel wall but also show evidence of adhesion to or invasion of surrounding structures, free perforation, obstruction, or aneuploidy. More importantly, recent data using molecular markers such as loss of heterozygosity (LOH) of chromosome 18q or the presence of microsatellite stable tumors, have helped to identify a subgroup of patients with both stage II and stage III CRC who may have much worse prognoses and in whom the administration of chemotherapy may be beneficial.

Tanaka et al, Clinical Cancer Research, 2011, discloses that the extent of CHFR promoter methylation correlates with recurrence free survival (RFS) in stage III but not in Stage II CRC patients.

Microsatellites are repeated sequences of DNA distributed throughout the genome. Although the length of these microsatellites is highly variable from person to person, each individual has microsatellites of a set length. These repeated sequences are common, and normal. The most common microsatellite in humans is a dinucleotide repeat of CA, which occurs tens of thousands of times across the genome.

In cells with mutations in DNA repair genes, however, some of these sequences accumulate errors and become longer or shorter. The appearance of abnormally long or short microsatellites in an individual's DNA is referred to as microsatellite instability. Microsatellite instability (MSI) is a condition manifested by damaged DNA due to defects in the normal DNA repair process. Sections of DNA called microsatellites, which consist of a sequence of repeating units of 1-6 base pairs in length, become unstable and can shorten or lengthen.

MSI is a key factor in several cancers including colorectal cancers. Colorectal cancer studies have demonstrated two mechanisms for MSI occurrence.

The first is in hereditary non-polyposis colorectal cancer (HNPCC) or Lynch Syndrome, where an inherited mutation in a mismatch-repair gene causes a microsatellite repeat replication error to go unfixed. The replication error results in a frame shift mutation that inactivates or alters major tumor suppressor genes - key genes in the regulation of the cell cycle and, ultimately, the prevention of cancer.

The second mechanism whereby MSI causes colorectal cancer is an epigenetic change that silences an essential mismatch-repair gene. In both cases, microsatellite insertions and deletions within tumor suppressor gene coding regions result in uncontrolled cell division and tumor growth.

Five markers have been recommended by the National Cancer Institute to screen for MSI in HNPCC tumors (often called Bethesda markers). Generally, MSI detection in two of the markers is considered a positive result or high probability of MSI (MSI-H).

The absence of microsatellite instability is termed microsatellite stability or MSS.

Over the last 15 years, the development of adjuvant chemotherapy given after surgical removal of tumors for patients with stage II disease has been used to reduce the risk of recurrence of cancer that may result from remaining tumor cells not detectable after surgery. Many thousands of patients with CRC have been included in clinical trials to assess the potential benefit of various combinations of chemotherapeutic agents.

Since the National Institutes of Health 1990 consensus conference, the administration of adjuvant 5-fluorouracil (FU)-based therapy for all medical patients with stage III colorectal cancer has become standard of care and has resulted in a 30%-40% decrease in relapse and mortality rates versus treatment with surgery alone. At the time, the panel did not recommend adjuvant therapy for stage II CRC patients outside the realm of clinical trials, as the data at that time did not support adjuvant therapy for stage II disease. However, one of the problems in the analysis of stage II disease has been the requirement for very large numbers of patients due to the overall favorable prognosis for this subgroup of patients. In adjuvant CRC studies, most clinical trials have included patients with both stage II and stage III disease, and most of those trials have been insufficiently powered to detect any treatment benefit in stage II patients.

Adjuvant chemotherapy is recommended only for stage III CRC patients. In Europe, the majority of stage II CRC patients undergo surgery alone, despite the recognition that a subgroup with a poor prognosis would probably benefit from adjuvant chemotherapy.

More reliable markers for differentiating between subgroups that may benefit from surgery alone and subgroups that require adjuvant therapy are desired.

### DISCLOSURE

The present invention relates to a method for determining the prognosis of a patient diagnosed with microsatellite stable, stage II colorectal cancer wherein the CpG island methylation status of the promoter of the CHFR gene is determined and wherein methylation of the promoter is indicative of a poor prognosis.

Within the group of patients diagnosed with stage II, microsatellite stable, colorectal cancer, we observed that the group with promoter CpG island methylation of the CHFR gene had a significantly poorer prognosis as compared to the group without CHFR promoter CpG island methylation.

This is clinically relevant since the group with a poorer prognosis may now be treated at an earlier stage of the disease with adjuvant therapy (such as adjuvant chemotherapy) whereas the group without CHFR promoter CpG island methylation may best be treated by surgery alone.

The invention, therefore, relates to a method for determining the prognosis of a patient diagnosed with microsatellite stable, stage II colorectal cancer wherein CpG island methylation status of the promoter of the CHFR gene is determined and wherein the presence of methylation of the promoter of the CHFR gene is indicative of a poor prognosis.

### BRIEF DESCRIPTION OF THE DRAWINGS

FIG. 1: The prognostic effect of CHFR methylation in 135 stage 2, MSS, BRAF wild-type CRC patients. CHFR unmethylated, upper line, CHFR methylate, lower line.
FIG. 2: Prognostic data of an overall population of 599 CRC patients (including all stages as well as MSI and MSS subtypes), CHFR unmethylated, upper line, CHFR methylate, lower line.

### MODE(S) FOR CARRYING OUT THE INVENTION

Hence, according to one embodiment of the invention, the CpG island methylation status of the promoter of the CHFR gene is determined in the genome of a patient diagnosed with microsatellite stable, stage II colorectal cancer wherein the presence of CpG island methylation of the promoter of the CHFR gene is indicative of a poor prognosis.

According to a further embodiment, the present invention relates to a method for determining the prognosis of a patient diagnosed with microsatellite stable, stage II colorectal cancer wherein the patient also carries a wild-type BRAF gene, which is often associated with MSI and a favorable prognosis. CHFR methylation, which is associated with MSI and BRAF mutations, however, also occurs in MSS, BRAF wild-type colorectal cancers and has a prognostic value in this subgroup of colorectal cancers.

According to a further embodiment, the present invention relates to a method for determining the prognosis of a patient diagnosed with microsatellite stable, stage II colorectal cancer, wherein the CpG island methylation status of the promoter of the CHFR gene is determined using Methylation Specific PCR.

According to a further embodiment, the present invention relates to a method for determining the prognosis of a patient diagnosed with microsatellite stable, stage II colorectal cancer, wherein for the Methylation Specific PCR, use is made of at least one primer pair selected from the group consisting of primer pair 1:
GAT TGT AGT TAT TTT TGT GAT TTG TAG GTG AT (SEQ ID NO:3) and
AAC TAA AAC AAA ACC AAA AAT AAC CCA CA (SEQ ID NO:4)
and primer pair 2:
GTT ATT TTC GTG ATT CGT AGG CGA C (SEQ ID NO:5) and
CGA AAC CGA AAA TAA CCC GCG (SEQ ID NO:6).

In a further embodiment, the invention relates to a method of treatment of a patient diagnosed with microsatellite stable, stage II colorectal cancer, wherein the methylation status of the CpG islands of the promoter of the CHFR gene of the patient is determined and the patient is treated with adjuvant therapy, preferably adjuvant chemotherapy.

### Definitions

Microsatellite instability (MSI) and conversably Microsatellite stability may be determined by a pentaplex PCR, using the mononucleotide MSI markers BAT-26, BAT-25, NR-21, NR-22 and NR-24, as previously described (N. Suraweera, A. Duval, M. Reperant, et al., Evaluation of tumor microsatellite instability using five quasi-monomorphic mononucleotide repeats and pentaplex PCR, Gastroenterology 123:1804-11, 2002). MSI was defined positive when three or more of five markers (BAT-26, BAT-25, NR-21, NR-22 and NR-24) showed allelic size variants.

With "stage II colorectal cancer" is meant the stage of colorectal cancer, wherein the tumor extends through the muscular wall of the colon, but wherein no metastasis in the lymph nodes is detected.

As used herein, with "determining the CpG island methylation status" is meant the determination of the methylation of the CpG islands, which may be accomplished using sodium bisulfite modification of genomic DNA. In a preferred embodiment, the level of methylation is determined. The higher the methylation level, the poorer the prognosis may become.

As used herein, with "CHFR gene" is meant the gene that encodes the checkpoint with forkhead and finger domains protein.

As used herein, with "promoter" is meant a region of DNA that facilitates the transcription of a particular gene.

As used herein, with "BRAF gene" is meant the gene that encodes the protein B-Raf (a serine/threonine-protein kinase).

As used herein, with "Methylation Specific PCR" is meant the PCR method, which is sensitive and specific for methylation of CpG sites in a CpG island, as described by J.G. Herman, J.R. Graff, and S. Myohanen, et al., Methylation-specific PCR: a novel PCR assay for methylation status of CpG islands, Proc. Natl. Acad. Sci. U. S. A. 93:9821-6, 1996.

### Examples

### Example 1: Study population

CRC patients were entered in two multi-center prospective clinical trials between 1979 and 1981 in the Netherlands. Tumor stage was defined according to the UICC-TNM staging system and American Joint Committee on Cancer classifications (AJCC), Cancer Staging Sixth Edition. Follow-up took place every three months during the first three years and every six months between three and five years after initial diagnosis and surgery. Standard protocols were followed, with routine blood counts and chemistry studies (including CEA levels) at each visit and liver ultrasound, chest x-ray and colonoscopy annually, to evaluate recurrence of disease and disease-related death. After five years of follow-up, only time and cause of death were registered. Follow-up was complete for all patients. Failure was defined as death due to recurrent disease, excluding postoperative mortality within 30 days and non-disease related death. For molecular analyses, tumor tissues from 173 patients with primary CRC were available. The distribution of age, sex, tumor location, stage, event frequency and mean follow-up time are provided in Table 1.

| **Table 1:** Clinicopathological characteristics of the CRC populations | | |
|---|---|---|
| | | |

| | **Study population (n=173)** | **Validation population (n=734)** |
|---|---|---|
| **Age** | | |
| Mean age (SD) | 67.8 (11.8) | 62.9 (4.1) |
| | | |
| **Gender** | | |
| Male | 82 (47%) | 408 (56%) |
| Female | 91 (53%) | 326 (44%) |
| | | |
| **Tumor location** | | |
| Right-sided colon | 62 (36%) | 239 (33%) |
| Left-sided colon | 52 (30%) | 310 (43%) |
| Rectum | 59 (34%) | 176 (24%) |
| | | |
| **CRC Stage** | | |
| I | 4 (2%) | 181 (27%) |
| II | 100 (58%) | 236 (35%) |
| III | 50 (29%) | 185 (28%) |
| IV | 19 (11%) | 69 (10%) |
| | | |
| **Event frequency**** | 64 (38%) | 302 (41%) |
| | | |
| **Median follow up time** | 4.8 years | 7.6 years |

| | | |
|---|---|---|
| SD: Standard Deviation ** colorectal cancer specific death | | |

### Example 2: Independent, population based series of CRCs

A second, independent population of 734 CRC cases, derived from the prospective Netherlands Cohort Study on diet and cancer, which started in 1986 with the enrolment of 120,852 healthy individuals between 55 and 69 years old from 204 municipalities throughout the Netherlands, was used to validate survival data. From 1989 until 1994, 925 incident CRC cases (ICD-O:153.0-154.1) were identified by computerized linkage with the Netherlands Cancer Registry and PALGA, a nationwide network and registry of histopathology and cytopathology (P.A. Van den Brandt, L.J. Schouten, R.A. Goldbohm, et al., Development of a record linkage protocol for use in the Dutch Cancer Registry for Epidemiological Research, Int. J. Epidemiol. 19:553-8, 1990). Information on tumor localization, tumor staging, differentiation grade and incidence date was available through the Netherlands Cancer Registry. Vital status until May 2005 was retrieved from the Central Bureau of Genealogy and the municipal population registries and could be obtained for all cases. Causes of death were retrieved through linkage with Statistics Netherlands. Paraffin-embedded tumor tissue was collected from 54 pathology registries; tissue blocks for 734 (90%) of the CRC cases contained sufficient DNA for analyses. Details of this cohort have been described previously (P.A. van den Brandt, R.A. Goldbohm, P. van't Veer, et al., A large-scale prospective cohort study on diet and cancer in The Netherlands, J. Clin. Epidemiol. 43:285-95, 1990). Clinical pathological characteristics are provided for both populations (Table 1). In the validation study, more left-sided tumors (43%) (p<0.01) and stage I tumors (27%) (p<0.001) were diagnosed and the median follow-up time was longer in the validation population: 7.6 years compared to 4.8 years (p<0.001). However, event frequencies were comparable between both studies, making the validation population suitable for validation of prognostic markers.

### Example 3: Promoter CpG island methylation and BRAF analysis

Genomic DNA was extracted from CRC tissues using PureGene^{™} Genomic DNA Isolation Kit (Gentra Systems) according to the manufacturer's protocol.

Promoter CpG island methylation of *checkpoint with forkhead and ring finger domains (CHFR*) was determined using sodium bisulfite modification of genomic DNA (EZ DNA methylation kit, ZYMO research Co., Orange, CA). To facilitate Methylation Specific PCR (MSP) analysis on DNA retrieved from formalin-fixed, paraffin-embedded tissue, nested Methylation Specific PCR (MSP) was performed as described elsewhere (J.G. Herman, J.R. Graff, S. Myohanen, et al., Methylation-specific PCR: a novel PCR assay for methylation status of CpG islands, Proc. Natl. Acad. Sci. U.S.A. 93:9821-6, 1996; and S. Derks, M.H. Lentjes, D.M. Hellebrekers, et al., Methylation-specific PCR unraveled, Cell Oncol. 26:291-9,2004).

Primers and PCR conditions were as follows:
CHFR flank up: TTT TYG TTT TTT TTG TTT TAA TAT AAT ATG G (SEQ ID NO:1),
CHFR flank down: CRC TCA CCA AAA ACR ACA ACT AAA AC (SEQ ID NO:2),
CHFR unmethylated sense: GAT TGT AGT TAT TTT TGT GAT TTG TAG GTG AT (SEQ ID NO:3),
CHFR unmethylated antisense: AAC TAA AAC AAA ACC AAA AAT AAC CCA CA (SEQ ID NO:4),
CHFR methylated sense: GTT ATT TTC GTG ATT CGT AGG CGA C (SEQ ID NO:5), and
CHFR methylated antisense: CGA AAC CGA AAA TAA CCC GCG (SEQ ID NO:6).

Microsatellite instability (MSI) was determined by a pentaplex PCR, using the mononucleotide MSI markers BAT-26, BAT-25, NR-21, NR-22 and NR-24, as previously described (N. Suraweera, A. Duval, M. Reperant, et al., Evaluation of tumor microsatellite instability using five quasimonomorphic mononucleotide repeats and pentaplex PCR, Gastroenterology 123:1804-11, 2002). MSI was defined positive when three or more of five markers (BAT-26, BAT-25, NR-21, NR-22 and NR-24) showed allelic size variants.

The common V600E *BRAF* mutation in exon 15 was analyzed by semi-nested PCR and subsequent RFLP analysis (M. Luchtenborg, M.P. Weijenberg, P.A. Wark, et al., Mutations in APC, CTNNB1 and K-ras genes and expression of hMLH1 in sporadic colorectal carcinomas from the Netherlands Cohort Study, BMC Cancer 5:160, 2005; and N.L. Sieben, G.M. Roemen, J. Oosting, et al., Clonal analysis favours a monoclonal origin for serous borderline tumours with peritoneal implants, J. Pathol. 210:405-11, 2006).

### Example 4: Data analysis

Differences between methylation-, clinicopathological- and molecular characteristics were determined by the Pearson Chi-Square and Fisher's exact test where appropriate. Kaplan-Meier curves were used to evaluate the relationship between promoter CpG island methylation and patient survival in the overall population and stratified for tumor stage, MSI and BRAF mutation status. Statistical differences between groups were assessed by use of the Log-rank test. The endpoint for analyses was overall survival starting from the day of surgery until the time of death due to CRC. Independent variables predicting survival were evaluated in a multivariate model using Cox Regression analyses. The Cox-regression model including CHFR promoter CpG island methylation, age, gender, tumor location, differentiation grade and TNM Stage were used to assess the prognostic influence of these variables. All P values were two sided and P values <0.05 were considered statistically significant. SPSS 15.0 and Stata 10.0 were used for data analyses.

### Example 5: Results

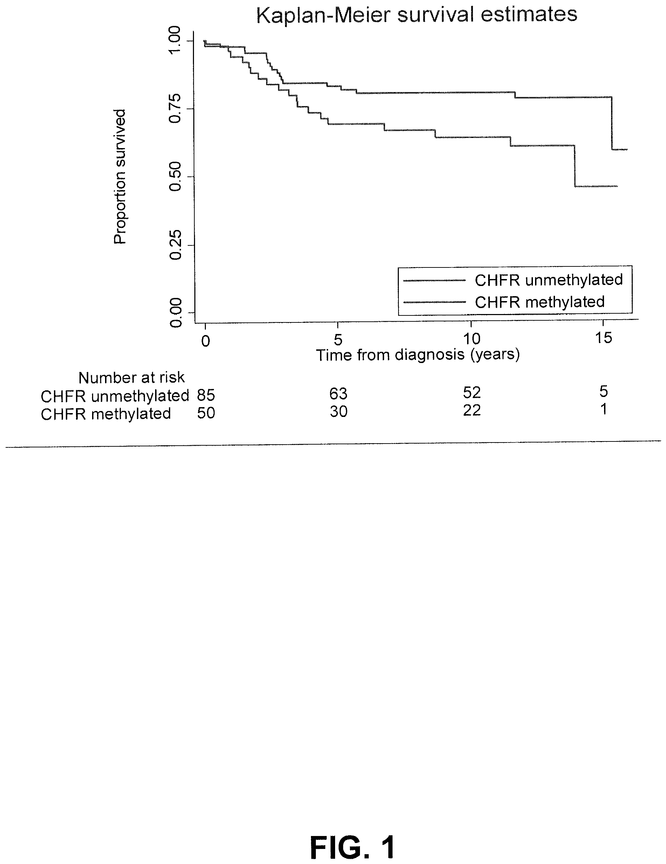

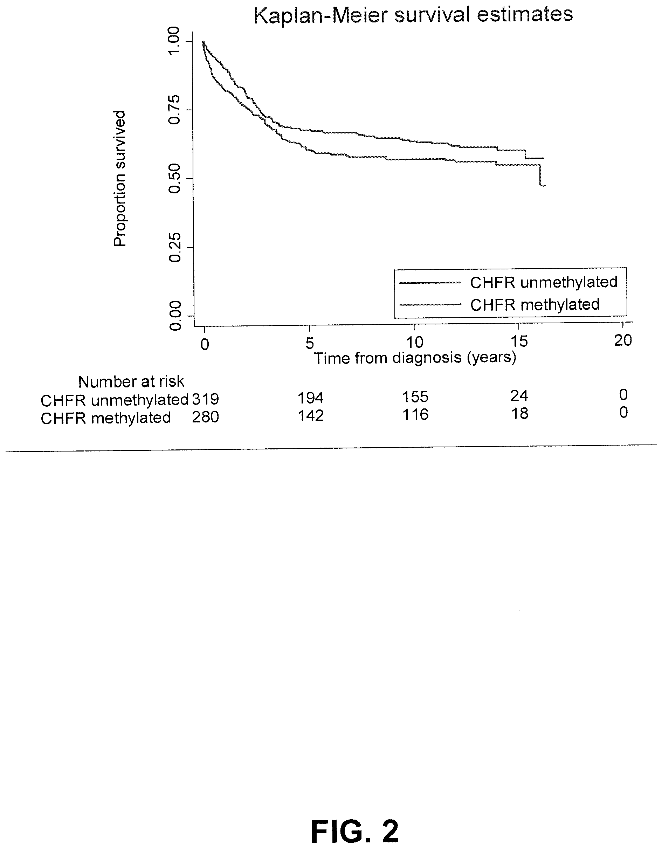

The prognostic effect of CHFR methylation in 135 stage 2, MSS, BRAF wild-type CRC patients is summarized in FIG. 1. In these studies, p=0.0269. Multivariate HR, adjusted for age at diagnosis, sex, differentiation grade and sublocation of the tumor: 2.36 (95%-CI 1.20-4.64, p=0.013). It should be noted that this effect is lost in the overall population of 599 CRC patients (including all stages as well as MSI and MSS subtypes). (FIG 2) In these studies, p=0.1116. Multivariate HR:1.09 (95%-CI 0.84-1.42, p=0.50).

In conclusion, we identified promoter CpG island methylation of CHFR as a prognostic biomarker in stage II, MSS, CRCs. This finding may be used to identify the subgroup of stage II CRC patients with an unfavourable prognosis that would benefit from adjuvant therapy, thereby preventing undertreatment of this patient group. In particular, the method according to the invention may be used to identify the subgroup of stage II BRAF wild-type CRC patients with a worse prognosis that would benefit from adjuvant therapy, thereby preventing undertreatment of this patient group.

| **List of abbreviations:** | |
|---|---|
| AJCC | American Joint Committee on Cancer |
| BRAF | Human gene encoding the serine/threonine-protein kinase B-Raf protein |
| CEA | Carcinoembryonic antigen |
| CHFR | Checkpoint with forkhead and ring finger domains |
| CIMP | CpG island methylator phenotype |
| CRC | Colorectal cancer |
| FU | Fluorouracil |
| HNPCC | Hereditary nonpolyposis colorectal cancer |
| LOH | Loss of heterozygosity |
| MI | Methylation Index |
| MSI | Microsatellite instability |
| MSP | Methylation specific PCR |
| MSS | Microsatellite stability |
| PCR | Polymerase chain reaction |
| RFLP | Restriction Fragment Length Polymerase |
| SSR | Simple sequence repeat |
| TNM | TNM Classification of Malignant Tumors |
| UICC | International Union Against Cancer |

## Claims

1. Method for determining the prognosis of a patient diagnosed with microsatellite stable, stage II colorectal cancer wherein the CpG island methylation status of the promoter of the CHFR gene is determined and wherein methylation of the promoter is indicative of a poor prognosis.

2. Method according to claim 1 wherein the patient also carries a wild-type BRAF gene.

3. Method according to claim 1 or 2, wherein the CpG island methylation status of the promoter of the CHFR gene is determined using Methylation Specific PCR.

4. Method according to claim 3, wherein at least one primer pair is used selected from the group consisting of primer pairs 1 (SEQ ID NO:3 and SEQ ID NO:4) and primer pair 2 (SEQ ID NO:4 and SEQ ID NO:6)

## Patentansprüche

1. Verfahren zur Bestimmung der Prognose für einen Patienten, bei dem mikrosatellitenstabiles Stadium-II-Kolorektalkarzinom diagnostiziert wurde, wobei der CpG-Insel-Methylierungsstatus des Promotors des CHFR-Gens bestimmt wird und wobei Methylierung des Promotors eine schlechte Prognose anzeigt.

2. Verfahren nach Anspruch 1, wobei der Patient auch ein Wildtyp-BRAF-Gen trägt.

3. Verfahren nach Anspruch 1 oder 2, wobei der CpG-Insel-Methylierungsstatus des Promotors des CHFR-Gens unter Verwendung von methylierungsspezifischer PCR (Methylation Specific PCR) bestimmt wird.

4. Verfahren nach Anspruch 3, wobei wenigstens ein Primer-Paar verwendet wird, das aus der aus Primer-Paar 1 (SEQ ID NO:3 und SEQ ID NO:4) und Primer-Paar 2 (SEQ ID NO:4 und SEQ ID NO:6) bestehenden Gruppe ausgewählt ist.

## Revendications

1. Procédé de détermination du pronostic d'un patient diagnostiqué avec un cancer colorectal de stade II à microsatellites stables dans lequel le statut de méthylation d'îlot CpG du promoteur du gène CHFR est déterminé et dans lequel la méthylation du promoteur est indicative d'un pronostic médiocre.

2. Procédé selon la revendication 1 dans lequel le patient comporte en outre un gène BRAF de type sauvage.

3. Procédé selon la revendication 1 ou 2, dans lequel l'état de méthylation d'îlot CpG du promoteur du gène CHFR est déterminé par PCR spécifique de la méthylation.

4. Procédé selon la revendication 3, dans lequel au moins une paire d'amorces est utilisée choisie dans le groupe constitué de la paire d'amorces 1 (SEQ ID NO: 3 et SEQ ID NO: 4) et la paire d'amorces 2 (SEQ ID NO: 4 et SEQ ID NO: 6).
